# EUROPÄISCHE PATENTSCHRIFT

(11) **EP 2 311 391 B1**
(45) Veröffentlichungstag und Bekanntmachung des Hinweises auf die Patenterteilung: **29.11.2017**
(21) Anmeldenummer: 10007704.9
(22) Anmeldetag: 24.07.2010
(51) Int. Cl.: A61B 17/225

(54) **Elektro-akustischer Wandler**
Electro-acoustic converter
Convertisseur électro-acoustique

(30) Priorität: 15.10.2009 DE 102009049487
(43) Veröffentlichungstag der Anmeldung: 20.04.2011
(73) Patentinhaber: Richard Wolf GmbH, 75438 Knittlingen (DE)
(72) Erfinder: Bauer, Edgar, 76703 Kraichtal (DE)
(74) Vertreter: Vollmann, Heiko

(56) Entgegenhaltungen:
- DE-A1- 19 624 443
- DE-C1- 19 543 741
- DE-C1- 19 733 233
- US-A- 4 617 931
- US-A1- 2003 026 435

## Beschreibung

Die Erfindung betrifft einen elektroakustischen Wandler, insbesondere zur Verwendung in einem Gerät zur medizinischen Stoßwellenbehandlung mit den im Oberbegriff des Anspruchs 1 angegebenen Merkmalen.

Derartige elektroakustische Wandler zählen zum Stand der Technik und werden beispielsweise in Lithotriptoren eingesetzt, beispielsweise zur nicht invasiven Behandlung von Steinleiden. Medizinische Stoßwellenbehandlungsgeräte sind inzwischen im Medizinbereich weit verbreitet und werden auch zu zahlreichen anderen Therapiezwecken eingesetzt. Es sind unterschiedliche physikalische Prinzipien zur Stoßwellenerzeugung bekannt. Gegenstand der vorliegenden Erfindung sind solche Geräte, die nach dem piezoelektrischen Prinzip arbeiten, das heißt, die einen elektroakustischen Wandler aufweisen, der aus einer Vielzahl von Piezoelementen aufgebaut ist.

Derartige elektroakustische Wandler sind häufig als selbstfokussierende Wandlerkalotten ausgebildet. Bei solchen Wandlerkalotten sind auf einer beispielsweise halbkugelförmigen Metallkalotte als Träger eine Vielzahl von Piezoelementen elektrisch leitend aufgeklebt und in eine elastische, elektrisch isolierende Vergussmasse aus Epoxidharz eingebettet. Der elektrische Anschluss der parallel zu schaltenden Piezoelemente erfolgt dabei einerseits über die metallische Kalotte und andererseits über eine Verdrahtung auf der anderen Seite der Piezoelemente. Ein so aufgebauter elektroakustischer Wandler ist beispielsweise aus DE 196 24 443 A1 bekannt.

Der elektroakustische Wandler bildet das Herzstück des Lithotriptors. Da er sehr aufwendig in der Herstellung und somit teuer ist, ist man bestrebt, diesen so aufzubauen, dass er neben guten therapeutischen Eigenschaften auch eine möglichst lange Lebensdauer aufweist. Die Lebensdauer eines solchen piezoelektrisch arbeitenden elektroakustischen Wandlers wird typischerweise durch zwei Ursachen begrenzt, zum einen durch Hochspannungsüberschläge an einem Piezoelement, welche das Element innerhalb kurzer Zeit zerstören und auch benachbarte Elemente sowie deren Isolation beschädigen und zum anderen dadurch, dass sich Piezoelemente von der Trägerkalotte lösen, was zunächst zu einem Leistungsabfall führt, jedoch beim Weiterbetreiben des Wandlers zu Rissbildung innerhalb des Piezoelements und schließlich zu einem Hochspannungsüberschlag führt.

Während die Hochspannungsüberschläge durch fehlerhafte Piezoelemente praktisch unabhängig von der Betriebszeit auftreten und eher Ausnahmen darstellen, ist das Sichlösen von Piezoelementen von der Trägerkalotte stark abhängig von der Betriebszeit des Wandlers.

Aus DE 196 24 443 A1 zählt es zwar zum Stand der Technik, durch eine spezielle Isolation an den Piezoelementen die Neigung zu einem Hochspannungsüberschlag zu vermindern, dies hat jedoch auf das Problem des Ablösens der Piezoelemente von der Trägerkalotte keinen Einfluss.

Die Befestigung der Piezoelemente auf der Trägerkalotte ist aus DE 34 25 992 A1 sowie DE 33 19 871 A1 bekannt. Sie erfolgt mittels eines elektrisch leitenden Epoxidklebers mit hohem Silberanteil, mit welchem die Piezoelemente elektrisch leitend und mechanisch mit der metallischen Trägerkalotte verbunden werden. Gerade in diesem Bereich erfolgen jedoch bei Langzeitbelastung die Ablösungen.

Vor diesem Hintergrund ist es Aufgabe der vorliegenden Erfindung, einen elektroakustischen Wandler, der aus Piezoelementen aufgebaut ist, so auszubilden, dass er einerseits mit hohem Wirkungsgrad arbeitet, andererseits jedoch langzeitstabil ist, das heißt eine hohe Lebensdauer aufweist.

Diese Aufgabe wird gemäß der Erfindung durch einen Wandler mit den in Anspruch 1 angegebenen Merkmalen gelöst. Vorteilhafte Ausgestaltungen der Erfindung sind in den Unteransprüchen, der nachfolgenden Beschreibung und der Zeichnung angegeben.

Der erfindungsgemäße elektroakustische Wandler, der insbesondere zur Verwendung in einem Gerät zur medizinischen Stoßwellenbehandlung vorgesehen und bestimmt ist, weist mehrere nebeneinander angeordnete Piezoelemente auf und ist dadurch gekennzeichnet, dass diese nebeneinander angeordneten Piezoelemente trägerlos angeordnet und in einer Verbundmasse eingebettet sind.

Die Piezoelemente sind dabei kräftefrei in der Verbundmasse eingegliedert. Durch die Flexibilität der Verbundmasse ist eine ausreichende Bewegungsfreiheit der Piezoelemente gewährleistet, wie sie bei Spannungsbeaufschlagung erforderlich ist.

Grundgedanke der vorliegenden Erfindung ist es, auf den typischerweise metallischen Träger des Wandlers zu verzichten und diesen trägerlos durch in eine Verbundmasse eingebettete Piezoelemente zu realisieren. Dadurch, dass die Piezoelemente trägerlos angeordnet sind, kann das Problem, dass sich diese vom Träger lösen, nicht mehr auftreten. Der mechanische Verbund wird durch das Einbetten in die Verbundmasse erreicht, wobei vorteilhaft eine vollständige Einbettung der Piezoelemente einschließlich der elektrischen Anschlüsse erfolgt. Dabei ist die Verbundmasse so zu wählen, dass sie einerseits einen ausreichend mechanischen Halt bietet, also eine gewisse Eigenstabilität des Wandlers gewährleistet, andererseits jedoch möglichst elastisch ist, um die Bewegung der Piezoelemente bei Spannungsbeaufschlagung schadenfrei zu überstehen. Die hierzu verwendeten Piezoelemente sind frei wählbar, typischerweise werden die gleichen oder ähnliche Piezoelemente eingesetzt, die auch beim Stand der Technik Verwendung finden, also im Wesentlichen zylindrische Piezoelemente, die an ihren voneinander abgewandten Stirnseiten kontaktiert werden.

Besonders vorteilhaft wird die Verbundmasse gemäß einer Weiterbildung der Erfindung durch eine elastische Vergussmasse gebildet, welche elektrisch isolierend ist und die Piezoelemente vorzugsweise allseitig umgibt. Eine solche elastische Vergussmasse kann vorteilhaft durch ein aushärtendes Epoxidharz gebildet sein, eine Einbettung in thermoplastische Kunststoffe ist ebenfalls möglich. Die Piezoelemente sind vorteilhaft mit Abstand zueinander angeordnet, wobei die Vergussmasse die dadurch gebildeten Zwischenräume ausfüllt.

Die Kontaktierung der Piezoelemente erfolgt bei dem erfindungsgemäßen elektroakustischen Wandler vorteilhaft über elektrisch leitende Verbindungsdrähte, die jeweils an den Stirnseiten der Piezoelemente kontaktiert sind und welche die Piezoelemente in an sich bekannter Weise elektrisch parallel schalten. Es ist also mindestens ein Leitungspaar zum Anschluss des elektroakustischen Wandlers aus der Verbundmasse herausgeführt. Die elektrische Kontaktierung mittels Verbindungsdrähten ist besonders vorteilhaft, wenn der Wandler zwei oder mehrlagig aufgebaut ist, da dann die benachbarten Lagen von Piezoelementen nicht wie beim Stand der Technik über die Trägerkalotte, sondern gesondert kontaktiert werden können, wodurch auch eine voneinander unabhängige elektrische Ansteuerung möglich ist, was von Vorteil ist, da dann der elektroakustische Wandler in besonders idealer Weise an den jeweiligen therapeutischen Zweck anpassbar ist.

Um zu verhindern, dass durch die elektrische Kontaktierung der Piezoelemente funktionale Einschränkungen eintreten, ist es gemäß einer Weiterbildung der Erfindung von Vorteil, möglichst dünne Verbindungsdrähte, typischerweise mit einem Durchmesser zwischen 0,1 mm bis 0,8 mm einzusetzen und diese ebenfalls in die Verbundmasse zusammen mit den Piezoelementen einzugliedern. Eine solche Drahtdicke ermöglicht bei den typischerweise für solche Wandler eingesetzten Piezoelementen einerseits eine ausreichende Stromversorgung, andererseits eine gute Beweglichkeit innerhalb der elastischen und flexiblen Verbundmasse. Um den bei höheren Frequenzen auftretenden Skineffekt zu vermindern, kann statt der vorgenannten Drahtdimensionierung auch eine Litze aus gewebten, feinen Metalldrähten verwendet werden.

Bei elektroakustischen Wandlern der Eingangs genannten Art, die einen metallischen Träger aufweisen, bildet der Träger ein so genanntes Backing, wodurch der Zuganteil der Schallwellen verringert werden kann, was beispielsweise bei der Steinzertrümmerung von Vorteil ist. Ein solches Backing ist gemäß einer vorteilhaften Weiterbildung der Erfindung auch im Zusammenhang mit dem erfindungsgemäßen elektroakustischen Wandler realisierbar, indem die nebeneinander angeordneten Piezoelemente jeweils mit einem eigenen Backing, also einer Rückseitenverstärkung versehen sind, wobei diese Rückseitenverstärkung ebenfalls in die Verbundmasse eingebettet ist, vorzugsweise zusammen mit den zugehörigen Piezoelementen und allseits mit Verbundmasse umgeben ist. Die erfindungsgemäße Rückseitenverstärkung erlaubt im Vergleich zum Stand der Technik eine wesentlich individuellere Konfiguration, da Stärke und Form des Backings praktisch beliebig gestaltet werden kann, anders als dies beim Stand der Technik der Fall ist.

Vorteilhaft wird die Rückseitenverstärkung durch jeweils an der rückseitigen Stirnseite des Piezoelements aufgeklebte Verstärkungskörper gebildet. Ein solcher Verstärkungskörper ist vorteilhaft elektrisch leitend, vorzugsweise aus Metall gebildet und elektrisch leitend mit einer Seite des zugehörigen Piezoelementes, nämlich der in Hauptabstrahlrichtung des Wandlers gesehenen Rückseite verbunden. Der elektrische Anschluss dieses Piezoelemente kann dann in vorteilhafter Weise über den Verstärkungskörper selbst erfolgen, vorzugsweise durch Kontaktierung an der vom Piezoelement abgewandten Seite des Verstärkungskörpers. Diese Ausbildung hat auch fertigungstechnisch Vorteile, da lediglich das jeweilige Piezoelement mit dem Verstärkungskörper zu verbinden ist, wonach die Gesamtheit dann zu einem elektroakustischen Wandler aufgebaut werden kann, wie dies bei Piezoelementen ohne Backing in gleicher Weise erfolgt.

Wenn, was gegebenenfalls auch vorteilhaft sein kann, der Verstärkungskörper aus einem elektrisch nicht leitenden Material, wie beispielsweise Glas, Keramik oder einem Verbundwerkstoff gebildet ist, dann ist es zweckmäßig, diesen mit einer elektrisch leitenden Schicht zu versehen, die dann elektrisch leitend mit dem zugehörigen Piezoelement verbunden ist. Der elektrische Anschluss des Piezoelementes erfolgt dann in gleicher Weise wie vorbeschrieben, nämlich dadurch, dass das Piezoelement an der Seite, an welcher der Verstärkungskörper anschließt, über die elektrisch leitende Schicht vorzugsweise an der vom Piezoelement abgewandten Seite des Verstärkungskörpers kontaktiert ist.

Durch Material und Formgebung des Verstärkungskörpers können die akustischen Eigenschaften des erfindungsgemäßen Wandlers in weiten Bereichen variiert werden. Besonders vorteilhaft ist der Verstärkungskörper durch eine zylindrische, kegel-, kegelstumpfförmige oder halbkugelförmige Form gebildet. Es können auch Kombinationen vorgesehen sein, beispielsweise ein zylindrischer Abschnitt, der an das Piezoelement anschließt und auf der abgewandten Seite halbkugelförmig ausgebildet ist.

Der erfindungsgemäße elektroakustische Wandler kann so ausgebildet sein, dass die nebeneinander angeordneten Piezoelemente in einer Ebene liegen (Planarwandler). Wenn die davon ausgehenden Schallwellen dann zu fokussieren sind, ist beispielsweise eine akustische Linse zu verwenden. Soll hingegen beispielsweise eine linienförmige Fokussierung erfolgen, so können die Piezoelemente in einer einachsig gekrümmten Raumform, beispielsweise in einem Zylindersegment angeordnet sein. Auch eine zweiachsig gekrümmte Raumform ist realisierbar, indem die Piezoelemente nach Art einer Kugelkalotte angeordnet werden, um eine fokussierte elektroakustische Wirkung zu erreichen, wie sie die Eingangs zum Stand der Technik beschriebenen elektroakustischen Wandler aufweisen.

Der erfindungsgemäße elektroakustische Wandler kann also in praktisch allen gewünschten Formen ausgebildet sein. Auch ist es gemäß einer Weiterbildung der Erfindung vorgesehen, Gruppen von nebeneinander angeordneten Piezoelementen mit Abstand hintereinander anzuordnen und in der Verbundmasse einzubetten. Besonders vorteilhaft sind dabei die Gruppen unabhängig voneinander elektrisch anschließbar, um so die einzelnen Gruppen beispielsweise zeitversetzt ansteuern zu können und die Schallwellen gezielt modulieren zu können. Es können damit elektroakustische Wandler geschaffen werden, die denen aus DE 197 33 233 C1 Bekannten in der Wirkweise ähneln.

Die Erfindung ist nachfolgend anhand von in der Zeichnung dargestellten Ausführungsbeispielen näher erläutert. Es zeigen:
- Fig. 1: in stark vereinfachter schematischer Darstellung den Aufbau eines erfindungsgemäßen Wandlers im Schnitt,
- Fig. 2: die Einzelheit II in Fig. 1,
- Fig. 3: eine andere Ausführungsform des Wandlers in Darstellung nach Fig. 1,
- Fig. 4: die Einzelheit IV in Fig. 3, und
- Fig. 5: eine dritte Ausführungsform in Darstellung nach Fig. 1

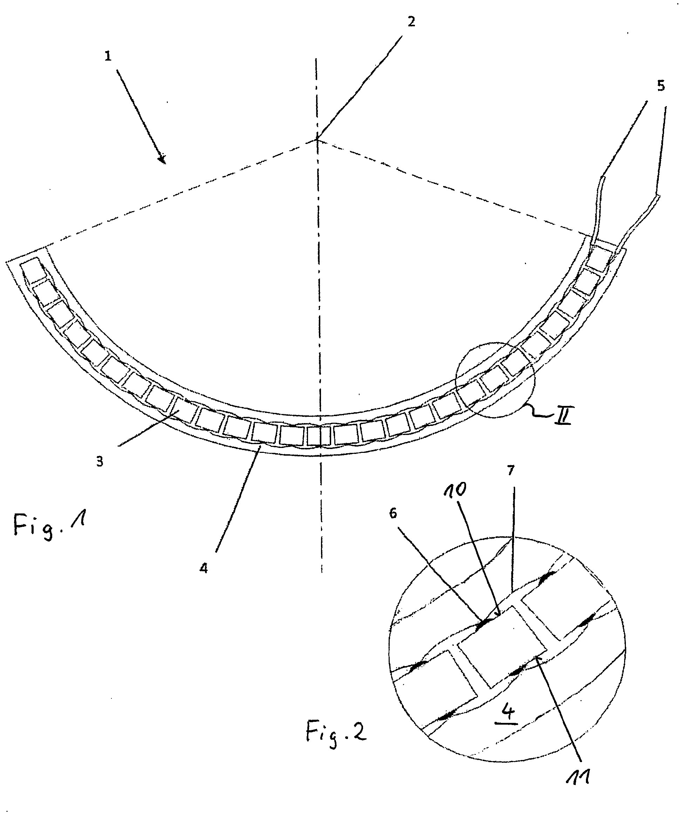

In Fig. 1 ist ein elektroakustischer Wandler 1 in Form einer selbst fokussierenden Wandlerkalotte dargestellt, wie sie bei Stoßwellenbehandlungsgeräten, die nach dem piezoelektrischen Prinzip arbeiten, häufig Verwendung findet. Der elektroakustische Wandler 1 weist einen Therapiefokus 2 auf, der durch den geometrischen Mittelpunkt der Wandlerkalotte 1 gebildet ist. Auch dieser elektroakustische Wandler 1 besteht aus einer Vielzahl mit geringem Abstand nebeneinander angeordneten Piezoelementen 3, die trägerlos so angeordnet sind, dass sie auf einer gedachten Kugelkalotte liegen. Gehalten werden die Piezoelemente 3 durch eine Verbundmasse 4 in Form einer flexiblen Vergussmasse, in welcher die Piezoelemente 3 vollständig, das heißt allseits und kräftefrei eingebettet sind. Die Vergussmasse 4 ist durch ein aushärtendes Epoxidharz gebildet und weist einerseits eine solche Flexibilität und Elastizität auf, dass die darin eingebetteten Piezoelemente 3 sich bei Spannungsbeaufschlagung entsprechend ausdehnen können, ohne die Vergussmasse 4 zu schädigen, andererseits jedoch so eigenstabil, dass sie die Piezoelemente 3 in der dargestellten Kalottenform hält.

Die Piezoelemente weisen eine im Wesentlichen zylindrische Form auf und haben eine zum Therapiefokus 2 des Wandlers 1 gerichtete Stirnseite 10 sowie eine davon abgewandte nach außen gerichtete Stirnseite 11. An den Stirnseiten 10 und 11 sind die Piezoelemente 3 kontaktiert und elektrisch angeschlossen. Jede Kontaktierung 6 an einer Stirnseite 10 bzw. 11 verbindet ein Piezoelement 3 mit einem elektrisch leitenden Draht 7. Mittels der Drähte 7 sind die nebeneinander angeordneten Piezoelemente 3 parallel geschaltet, das heißt, die Kontaktierungsstellen 6 an den nach innen gerichteten Stirnseiten 10 sind einerseits elektrisch leitend verbunden und die Kontaktierungsstellen 6 an den nach außen weisenden Stirnseiten 11 sind andererseits elektrisch leitend verbunden und seitlich als elektrische Anschlüsse 5 aus dem Wandler 1, insbesondere der Vergussmasse 4 herausgeführt. Über die Anschlüsse 5 können die nebeneinander angeordneten Piezoelemente 3 mit Spannung beaufschlagt werden, eben zum Erzeugen von Stoßwellen.

Die elektrischen Drähte 7, welche die Piezoelemente 3 elektrisch miteinander verbinden, weisen einen Durchmesser zwischen 0,1 mm und 0,8 mm auf, sind also so dimensioniert, dass sie einerseits einen ausreichenden Leitungsquerschnitt aufweisen, andererseits jedoch die Bewegung der Piezoelemente 3 bei Spannungsbeaufschlagung möglichst wenig behindern. Anstelle von Einzeldrähten können auch geflochtene Litzen oder dergleichen eingesetzt werden, die hochflexibel sind. Eine solche Anordnung ist insbesondere bei Ansteuerung des Wandlers mit höheren Frequenzen zu empfehlen, um einem möglichen Skineffekt entgegenzuwirken.

Das anhand von Fig. 1 und 2 dargestellte und vorbeschriebene Ausführungsbeispiel zeigt einen Wandler in Kalottenform, es versteht sich jedoch, dass dies nur eine Prinzipdarstellung ist und dass der Wandler 1 in praktisch jeder beliebigen Raumform sowie auch als Planarwandler nach dem vorbeschriebenen Bauprinzip ausgebildet werden kann.

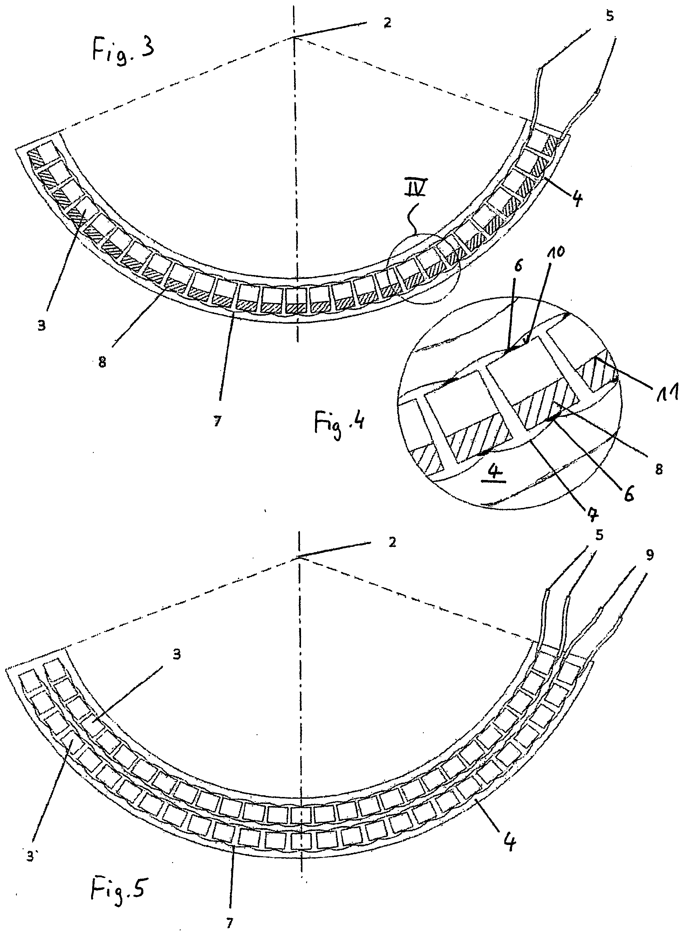

Anhand der Fig. 3 und 4 ist eine andere Ausführung eines Wandlers dargestellt, bei dem quasi ein Backing vorgesehen ist, wie dies bei Wandlerkalotten mit Träger nach dem Stand der Technik an sich bekannt ist. Das Backing ist hier durch eine Rückseitenverstärkung jedes einzelnen Piezoelementes 3, nämlich durch einen an der nach außen weisenden Stirnseite 11, also der Rückseite jedes Piezoelementes 3 angebrachten Verstärkungskörper 8 gebildet. Die Verstärkungskörper 8 weisen im dargestellten Ausführungsbeispiel Zylinderform auf und sind hinsichtlich ihrer Umfangskontur an die der Piezoelemente 3 angepasst. Die Verstärkungskörper 8 bestehen aus elektrisch leitendem Metall und sind an der rückseitigen Stirnseite 11 der Piezoelemente 3 elektrisch leitend angeklebt. Die Kontaktierung erfolgt, wie aus Fig. 4 ersichtlich ist, über Kontaktierungsstellen 6, die an der freien Stirnseite der Verstärkungskörper 8 angeordnet sind. Die Piezoelemente 3 werden also durch den Verstärkungskörper 8 in Achsrichtung verlängert, die Kontaktierung erfolgt dann an den beiden Stirnseiten des so gebildeten Zylinderkörpers, bestehend aus Piezoelement 3 und Verstärkungskörper 8.

Piezoelemente 3 und Verstärkungskörper 8 einschließlich Kontaktierung und Verdrahtung sind in der Vergussmasse 4 eingebettet. Um einen innigen Verbund zwischen der Vergussmasse 4 und den Verstärkungskörpern 8 zu erzielen, können die Verstärkungskörper 8 umfangseitig profiliert ausgebildet sein, beispielsweise durch nuten- oder wellenförmige Einstiche oder andere geeignete Oberflächenstrukturen. Die Verstärkungskörper 8 sind hier nur beispielhaft als Zylinderkörper ausgebildet, es könne auch andere geeignete Körperformen gewählt werden, zum Beispiel Kegelstumpf- oder Kalottenform, gegebenenfalls auch kombiniert, je nach Anforderung an das Stoßwellenprofil des Wandlers.

In Fig. 5 ist schematisch ein Mehrschichtenwandler 1 dargestellt. Dort sind in zwei bezogen auf den Fokus 2 hintereinander liegenden Schichten Gruppen von nebeneinander angeordneten Piezoelementen 3 in die Vergussmasse 4 eingebettet. Anschluss und Aufbau erfolgt analog zu der anhand der Fig. 1 und 2 beschriebenen Ausführung. Der in Fig. 5 dargestellte Wandler ist zweischichtig aufgebaut, weist also zwei Gruppen von parallel geschalteten Piezoelementen 3 auf, deren elektrische Anschlüsse gesondert herausgeführt sind, nämlich zu dem Anschlusspaar 5, welches die innenliegende Piezoelementgruppe anschließt und zu einem Anschlusspaar 9, welches die außenliegende Piezoelementgruppe anschließt.

Diese Anordnung hat gegenüber dem vergleichbaren Stand der Technik (DE 197 33 233 C1) den Vorteil, dass die Gruppen jeweils für sich elektrisch ansteuerbar sind und keinen gemeinsamen Anschluss haben, wie dies beim Stand der Technik durch den metallischen Kalottenkörper gebildet ist.

Die vorstehend beschriebenen Ausführungsbeispiele verdeutlichen den grundsätzlichen Aufbau des erfindungsgemäßen Wandlers, es versteht sich, dass eine Vielzahl unterschiedlicher Wandlerbauarten nach diesem Prinzip gebildet werden kann, die hier nicht im Einzelnen beschrieben sind.

### Bezugszeic henliste

- 1: - Wandler
- 2: - Therapiefokus
- 3: - Piezoelemente
- 4: - Vergussmasse
- 5: - Anschlüsse, Anschlusspaar
- 6: - Kontaktierungsstellen
- 7: - Drähte
- 8: - Verstärkungskörper
- 9: - Anschlüsse, Anschlusspaar
- 10: - innere Stirnseite des Piezoelementes
- 11: - äußere Stirnseite des Piezoelementes

## Patentansprüche

1. Elektroakustischer Wandler, insbesondere zur Verwendung in einem Gerät zur medizinischen Stoßwellenbehandlung, mit mehreren nebeneinander angeordneten Piezoelementen (3), **dadurch gekennzeichnet, dass** die Piezoelemente (3) trägerlos angeordnet und in einer Verbundmasse (4) eingebettet sind.

2. Elektroakustischer Wandler nach Anspruch 1, **dadurch gekennzeichnet, dass** die Verbundmasse (4) durch eine elastische Vergussmasse gebildet ist, welche elektrisch isolierend ist und die Piezoelemente (3) vorzugsweise allseitig umgibt.

3. Elektroakustischer Wandler nach Anspruch 1 oder 2, **dadurch gekennzeichnet, dass** die Piezoelemente (3) über diese stirnseitig kontaktierende elektrisch leitende Verbindungsdrähte (7) angeschlossen sind, wobei nebeneinander angeordnete Piezoelemente (3) parallel geschaltet sind.

4. Elektroakustischer Wandler nach einem der vorhergehenden Ansprüche, **dadurch gekennzeichnet, dass** die Verbindungsdrähte (7) einen Durchmesser zwischen 0,1 mm bis 0,8 mm aufweisen und in der Verbundmasse (4) eingegliedert sind.

5. Elektroakustischer Wandler nach einem der vorhergehenden Ansprüche, **dadurch gekennzeichnet, dass** nebeneinander angeordnete Piezoelemente (3) jeweils mit einer Rückseitenverstärkung (8) versehen sind, die ebenfalls in die Verbundmasse (4) eingebettet, vorzugsweise zusammen mit dem zugehörigen Piezoelement (3) allseits mit Verbundmasse (4) umgeben ist.

6. Elektroakustischer Wandler nach einem der vorhergehenden Ansprüche, **dadurch gekennzeichnet, dass** eine Rückseitenverstärkung (8) durch einen an der rückseitigen Stirnseite (11) des Piezoelements (3) aufgeklebten Verstärkungskörper (8) gebildet ist.

7. Elektroakustischer Wandler nach einem der vorhergehenden Ansprüche, **dadurch gekennzeichnet, dass** ein Verstärkungskörper (8) elektrisch leitend, vorzugsweise aus Metall gebildet ist, elektrisch leitend mit einer Seite des zugehörigen Piezoelements (3) verbunden ist und dass der elektrische Anschluss des Piezoelements (3) an dieser Seite über den Verstärkungskörper (8) vorzugsweise an der vom Piezoelement (3) abgewandten Seite des Verstärkungskörpers (8) erfolgt.

8. Elektroakustischer Wandler nach einem der vorhergehenden Ansprüche, **dadurch gekennzeichnet, dass** ein Verstärkungskörper (8) aus Glas, Keramik oder dgl. nichtleitendem Material gebildet ist und mit einer elektrisch leitenden Schicht versehen ist, die elektrisch leitend mit dem zugehörigen Piezoelement (3) verbunden ist, und dass der elektrische Anschluss des Piezoelements (3) an dieser Seite über die elektrisch leitende Schicht des Verstärkungskörpers (8), vorzugsweise an der vom Piezoelement (3) abgewandten Seite des Verstärkungskörpers (8) erfolgt.

9. Elektroakustischer Wandler nach einem der vorhergehenden Ansprüche, **dadurch gekennzeichnet, dass** ein Verstärkungskörper (8) eine zylindrische, kegel-, kegelstumpfförmige oder halbkugelförmige Form aufweist.

10. Elektroakustischer Wandler nach einem der vorhergehenden Ansprüche, **dadurch gekennzeichnet, dass** nebeneinander angeordnete Piezoelemente (3) in einer Ebene, in einer einachsig oder einer zweiachsig gekrümmten Raumform angeordnet sind.

11. Elektroakustischer Wandler nach einem der vorhergehenden Ansprüche, **dadurch gekennzeichnet, dass** Gruppen von nebeneinander angeordneten Piezoelementen (3) mit Abstand hintereinander angeordnet und in der Verbundmasse (4) eingebettet sind, wobei die Gruppen unabhängig voneinander elektrisch anschließbar sind.

## Claims

1. An electroacoustic transducer, in particular for use in an apparatus for medical shock-wave treatment, with several piezoelements (3) arranged next to one another, **characterised in that** the piezoelements (3) are arranged in a carrier-less manner and are embedded in a composite mass (4).

2. An electroacoustic transducer according to claim 1, **characterised in that** the composite mass (4) is formed by an elastic cast mass, which is electrically insulating and surrounds the piezoelements (3) preferably on all sides.

3. An electroacoustic transducer according to claim 1 or 2, **characterised in that** the piezoelements (3) are connected via electrically conductive connection wires (7) which contact these on the end-side, wherein piezoelements (3) arranged next to one another are connected in parallel.

4. An electroacoustic transducer according to one of the preceding claims, **characterised in that** the connection wires (7) have a diameter between 0.1 mm to 0.8 mm and are integrated in the composite mass (4).

5. An electroacoustic transducer according to one of the preceding claims, **characterised in that** piezoelements (3) arranged next to one another are provided in each case with a rear-side reinforcement (8), which is likewise embedded into the cast mass (4), preferably together with the associated piezoelement (3) is surrounded on all sides with composite mass (4).

6. An electroacoustic transducer according to one of the preceding claims, **characterised in that** a rear-side reinforcement (8) is formed by a reinforcement body (8), which is bonded on the rear-side end-side (11) of the piezoelement (3).

7. An electroacoustic transducer according to one of the preceding claims, **characterised in that** a reinforcement body (8) is electrically conductive, preferably is formed from metal and connected to one side of the associated piezoelement (3) in an electrically conductive manner, and that the electrical connection of the piezoelement (3) is effected at this side via the reinforcement body (8), preferably at the side of the reinforcement body (8) which is away from the piezoelement (3).

8. An electroacoustic transducer according to one of the preceding claims, **characterised in that** a reinforcement body (8) is formed of glass, ceramic or suchlike non-conductive material and is provided with an electrically conductive layer, which is connected to the associated piezoelement (3) in an electrically conductive manner and that the electrical connection of the piezoelement (3) at this side is effected via the electrically conductive layer of the reinforcement body (8), preferably at the side of the reinforcement body (8), which is away from the piezoelement (3).

9. An electroacoustic transducer according to one of the preceding claims, **characterised in that** a reinforcement body (8) has a cylindrical, conical, truncated cone shape or hemispherical shape.

10. An electroacoustic transducer according to one of the preceding claims, **characterised in that** piezoelements (3) arranged next to one another are arranged in a plane, in a space shape curved in a single-axis manner, or curved in a two-axis manner.

11. An electroacoustic transducer according to one of the preceding claims, **characterised in that** groups of piezoelements (3) arranged next to one another are arranged behind one another at a distance and are embedded in the composite mass (4), wherein the groups may be electrically connected independently of one another.

## Revendications

1. Convertisseur électro-acoustique, notamment pour une utilisation dans un appareil pour le traitement médical par ondes de choc, avec plusieurs éléments piézoélectriques (3) disposés les uns à côté des autres, **caractérisé en ce que** les éléments piézoélectriques (3) sont disposés sans support et sont incorporés dans une masse composite (4).

2. Convertisseur électro-acoustique selon la revendication 1, **caractérisé en ce que** la masse composite (4) est constituée par une masse coulée élastique qui est électriquement isolante et qui entoure les éléments piézoélectriques (3) de préférence de tous les côtés.

3. Convertisseur électro-acoustique selon la revendication 1 ou 2, **caractérisé en ce que** les éléments piézoélectriques (3) sont raccordés par des fils de connexion (7) électriquement conducteurs qui sont en contact avec ceux-ci sur la face avant de ceux-ci, des éléments piézoélectriques (3) disposés les uns à côté des autres étant raccordée en parallèle.

4. Convertisseur électro-acoustique selon l'une des revendications précédentes, **caractérisé en ce que** les fils de connexion (7) ont un diamètre entre 0,1 mm à 0,8 mm et sont incorporés dans la masse composite (4).

5. Convertisseur électro-acoustique selon l'une des revendications précédentes, **caractérisé en ce que** des éléments piézoélectriques (3) disposés les uns à côté des autres sont pourvus chacun d'un renforcement de face arrière (8) qui est également incorporé dans la masse composite (4) et est de préférence entouré, ensemble avec l'élément piézoélectrique associé (3), de tous les côtés de la masse composite (4).

6. Convertisseur électro-acoustique selon l'une des revendications précédentes, **caractérisé en ce qu'**un renforcement de face arrière (8) est constitué par un corps de renforcement (8) collé sur la face frontale (11) du côté arrière de l'élément piézoélectrique (3).

7. Convertisseur électro-acoustique selon l'une des revendications précédentes, **caractérisé en ce qu'**un corps de renforcement (8) est réalisé électriquement conducteur, de préférence en métal, qu'il est raccordé de façon électriquement conductrice à un côté de l'élément piézoélectrique (3) associé et que le raccordement électrique de l'élément piézoélectrique (3) est réalisé sur ce côté via le corps de renforcement (8), de préférence sur le côté du corps de renforcement (8) opposé à l'élément piézoélectrique (3).

8. Convertisseur électro-acoustique selon l'une des revendications précédentes, **caractérisé en ce qu'**un corps de renforcement (8) est réalisé en verre, en céramique ou en un matériau semblable non conducteur et est pourvu d'une couche électriquement conductrice qui est raccordée de façon électriquement conductrice à l'élément piézoélectrique (3) correspondant, et **en ce que** le raccordement électrique de l'élément piézoélectrique (3) est réalisé sur ce côté via la couche électriquement conductrice du corps de renforcement (8), de préférence sur le côté du corps de renforcement (8) qui est opposé à l'élément piézoélectrique (3).

9. Convertisseur électro-acoustique selon l'une des revendications précédentes, **caractérisé en ce qu'**un corps de renforcement (8) présente une forme cylindrique, conique, tronconique ou hémisphérique.

10. Convertisseur électro-acoustique selon l'une des revendications précédentes, **caractérisé en ce que** des éléments piézoélectriques (3) disposés les uns à côté des autres sont disposés dans un plan ou dans une forme tridimensionnelle courbée à un ou à deux axes.

11. Convertisseur électro-acoustique selon l'une des revendications précédentes, **caractérisé en ce que** des groupes d'éléments piézoélectriques (3) disposés les uns à côté des autres, sont disposés de façon espacé les uns derrière les autres et sont incorporés dans la masse composite (4), les groupes étant adaptés pour pouvoir être raccordés électriquement de façon indépendante les uns des autres.
